(19) Europäisches Patentamt / European Patent Office / Office européen des brevets

(11) **EP 4 035 739 A1**

(12) **EUROPEAN PATENT APPLICATION**
published in accordance with Art. 153(4) EPC

(43) Date of publication:
**03.08.2022 Bulletin 2022/31**

(21) Application number: **20870086.4**

(22) Date of filing: **16.07.2020**

(51) International Patent Classification (IPC):
*A61Q 19/00* $^{(2006.01)}$   *C08K 5/053* $^{(2006.01)}$
*C08K 7/02* $^{(2006.01)}$   *C08L 33/26* $^{(2006.01)}$
*C08L 39/06* $^{(2006.01)}$   *C08F 2/44* $^{(2006.01)}$
*A61K 9/70* $^{(2006.01)}$   *A61K 8/04* $^{(2006.01)}$
*A61K 8/34* $^{(2006.01)}$   *A61K 8/81* $^{(2006.01)}$

(52) Cooperative Patent Classification (CPC):
**A61K 8/04; A61K 8/34; A61K 8/81; A61K 9/70;
A61Q 19/00; C08F 2/44; C08K 5/053; C08K 7/02;
C08L 33/26; C08L 39/06**

(86) International application number:
**PCT/JP2020/027635**

(87) International publication number:
**WO 2021/059689 (01.04.2021 Gazette 2021/13)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **27.09.2019 JP 2019177030**

(71) Applicant: **Sekisui Kasei Co., Ltd.
Kita-ku
Osaka-shi
Osaka 530-8565 (JP)**

(72) Inventors:
• **IIZUKA, Ryo
  Osaka-shi, Osaka 530-8565 (JP)**
• **KATO, Kazuki
  Osaka-shi, Osaka 530-8565 (JP)**

(74) Representative: **Müller-Boré & Partner
Patentanwälte PartG mbB
Friedenheimer Brücke 21
80639 München (DE)**

(54) **HYDROGEL**

(57)    A hydrogel comprising a polymer matrix formed from a copolymer of an acrylic monomer and a crosslinkable monomer, a water-soluble polymer, water, and a polyhydric alcohol, the hydrogel having a tensile strength of 0.5 N/20 mm to 3.0 N/20 mm.

EP 4 035 739 A1

**Description**

Technical Field

The present invention relates to a hydrogel.

Background Art

Hydrogels are basically highly hydrophilic polymers swelling in an aqueous solvent. Hydrogels have various properties such as water absorbency, swelling, moisture retention, adhesion, and conductivity, depending on their use. Taking advantage of these properties, hydrogels have been used in a wide range of fields such as civil engineering and construction, agriculture, food, medical care, cosmetics, and electricity.

For example, Patent Literature (PTL) 1 discloses an adhesive gel (hydrogel having adhesion) containing a polyhydric alcohol and water in a matrix of a hydrophilic polymer and an acrylamide-based polymer. This adhesive gel can be suitably applied to a surface of a living body while maintaining its adhesion even when the water content decreases. The adhesive gel also has properties such as low skin irritation and flexibility.

PTL 2 discloses an adhesive gel containing a non-crosslinked water-soluble polymer having a weight average molecular weight of 4000 to 15000000 and water in a polymer matrix obtained by copolymerizing acrylamide as a polymerizable monomer and a crosslinkable monomer, wherein the gel contains the water-soluble polymer in an amount of 0.1 to 5.0 mass%, the adhesive force of the gel to 12 mm$\varphi$ stainless steel is 1.96 to 19.6 N, and the 90 degree peel strength of the gel to a biaxially oriented polyester film is 0.196 to 1.96 N/0.02 m. This adhesive gel has low skin irritation and also has low film peel strength with respect to a biaxially oriented polyester film and stable releasability while having high adhesion.

Citation List

Patent Literature

   PTL 1: JP2012-107120A

   PTL 2: JP2003-096431A

Summary of Invention

Technical Problem

Hydrogels preferably have less odor, less skin irritation, and flexibility when they are used for application to the skin. Moreover, it is common for hydrogels to be used repeatedly. It is thus preferable that the adhesion of a hydrogel when it is used repeatedly (which hereinafter may be simply referred to as "adhesion in repeated use") is high in order to maintain the adhesion to the skin surface in re-application. Neither PTL 1 nor PTL 2 addresses improvement in the adhesion of gels in repeated use.

Conventional hydrogels have insufficient adhesion in repeated use because their adhesive force decreases when application and peeling off are repeated several times. However, if acrylic acid is used in the polymer matrix to improve the adhesive force, the odor of the gel becomes stronger, which may cause discomfort to the user.

For repeated use, the followability to the skin can be increased by adjusting the hardness of the hydrogel. However, if the hydrogel is too soft, the hydrogel may tear during use.

An object of the present invention is to provide a hydrogel that has excellent adhesion in repeated use and is resistant to tearing.

Solution to Problem

The present inventors found that the above problems can be solved by selecting components of a hydrogel and setting the tensile strength in a specific range.

According to a first aspect of the present invention, there is provided a hydrogel comprising a polymer matrix formed from a copolymer of an acrylic monomer and a crosslinkable monomer, a water-soluble polymer, water, and a polyhydric alcohol, the hydrogel having a tensile strength of 0.5 N/20 mm to 3.0 N/20 mm.

According to a second aspect of the present invention, there is provided a method for producing the hydrogel, comprising irradiating a polymerizable solution with ultraviolet light having a peak intensity of 70 to 150 mW/cm$^2$, the polymerizable solution comprising an acrylic monomer, a crosslinkable monomer, a water-soluble polymer, water, and a polyhydric alcohol and having a crosslinkable monomer content of 0.03 mass% to 0.05 mass%.

Advantageous Effects of Invention

The present invention can provide a hydrogel that has excellent adhesion in repeated use and is resistant to tearing.

Brief Description of Drawings

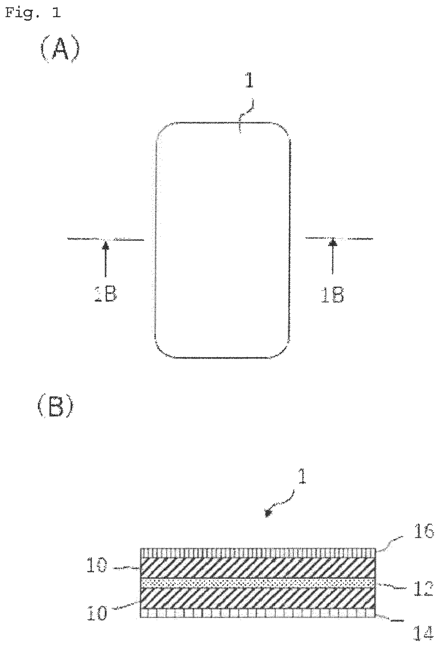

Fig. 1 (A) is a schematic plan view of an embodiment of a gel sheet. Fig. 1 (B) is a schematic cross-sectional view of the gel sheet of Fig. 1 (A) taken along the line 1B-1B in Fig. 1 (A) .

Description of Embodiments

Hydrogel

The hydrogel of the present invention (which hereinafter may be simply referred to as "hydrogel" or "gel") comprises a polymer matrix formed from a copolymer of an acrylic monomer and a crosslinkable monomer, a water-soluble polymer, water, and a polyhydric alcohol, and has a tensile strength of 0.5 N/20 mm to 3.0 N/20 mm.

The acrylic monomer is a general term for a monomer that contains acryloyl ($H_2C=CH-C(=O)-$) or methacryloyl ($H_2C=C(CH_3)-C(=O)-$) and is capable of forming a polymer by polymerization. The acrylic monomer is a monofunctional, non-crosslinkable monomer that contains one polymerizable carbon-carbon double bond in the molecule.

The acrylic monomer is not particularly limited and is preferably a water-soluble monomer such as a (meth)acrylamide-based monomer or a (meth)acrylic acid ester, and more preferably a (meth)acrylamide-based monomer.

Specific examples of (meth)acrylamide-based monomers include (meth)acrylamide; N,N-dialkyl(meth)acrylamides, such as N,N-dimethyl(meth)acrylamide and N,N-diethyl(meth)acrylamide; N-alkyl(meth)acrylamides, such as N-isopropyl(meth)acrylamide, N-methyl(meth)acrylamide, N-ethyl(meth)acrylamide, and N-propyl(meth)acrylamide; N-hydroxyalkyl(meth)acrylamides, such as N-hydroxyethyl(meth)acrylamide and N-hydroxymethyl(meth)acrylamide; N-alkoxyalkyl(meth)acrylamides, such as N-ethoxymethyl(meth)acrylamide, N-propoxymethyl(meth)acrylamide, N-butoxymethyl(meth)acrylamide, N-isobutoxymethyl(meth)acrylamide, N-pentoxymethyl(meth)acrylamide, N-hexyloxymethyl(meth)acrylamide, N-heptoxymethyl(meth)acrylamide, N-octoxymethyl(meth)acrylamide, N-ethoxyethyl(meth)acrylamide, N-propoxyethyl(meth)acrylamide, and N-butoxyethyl(meth)acrylamide; amino group-containing cationic acrylamide compounds, such as dimethylaminopropyl(meth)acrylamide; sulfonic group-containing anionic acrylic monomers, such as 4-acryloylmorpholine and tert-butyl acrylamide sulfonic acid, or salts thereof; and derivatives of these. Of these, one or more members selected from the group consisting of (meth)acrylamide, N,N-dimethyl(meth)acrylamide, N,N-diethyl(meth)acrylamide, N-isopropyl(meth)acrylamide, N-methyl(meth)acrylamide, N-ethyl(meth)acrylamide, N-propyl(meth)acrylamide, N-hydroxyethyl(meth)acrylamide, N-hydroxymethyl(meth)acrylamide, dimethylaminopropyl(meth)acrylamide, 4-acryloylmorpholine, and tert-butyl acrylamide sulfonic acid and salts thereof are preferred; however, the (meth)acrylamide-based monomer is not limited thereto.

Specific examples of (meth)acrylic acid esters include, but are not limited to, one or more members selected from the group consisting of (meth)acrylic acid alkyl esters having an alkyl group with 1 to 18 carbon atoms, such as methyl (meth)acrylate, ethyl (meth)acrylate, n-propyl (meth)acrylate, isopropyl (meth)acrylate, n-butyl (meth)acrylate, isobutyl (meth)acrylate, tert-butyl (meth)acrylate, n-hexyl (meth)acrylate, n-octyl (meth)acrylate, isooctyl (meth)acrylate, 2-ethylhexyl (meth)acrylate, n-nonyl (meth)acrylate, isononyl (meth)acrylate, n-pentyl (meth)acrylate, n-decyl (meth)acrylate, isodecyl (meth)acrylate, n-lauryl (meth)acrylate, tridecyl (meth)acrylate, and n-stearyl (meth)acrylate; alicyclic (meth)acrylic acid esters, such as cyclohexyl (meth)acrylate, isobornyl (meth)acrylate, and 1-adamantyl (meth)acrylate; alkoxy group-containing (meth)acrylic acid esters, such as 2-methoxyethyl (meth)acrylate, ethoxyethoxyethyl (meth)acrylate, and methoxypolyethyleneglycol (meth)acrylates (e.g., methoxytriethyleneglycol (meth)acrylate); hydroxyalkyl (meth)acrylates (in which an aryl group may be bonded to the hydroxyalkyl group via an ether linkage), such as 2-hydroxyethyl (meth)acrylate, 2-hydroxypropyl (meth)acrylate, 2-hydroxy-3-phenoxypropyl (meth)acrylate, and 2-hydroxybutyl (meth)acrylate; glycerol mono(meth)acrylate; polyalkylene glycol mono(meth)acrylates, such as polyethylene glycol mono(meth)acrylate and a polyethylene glycol-polypropylene glycol copolymer; (meth)acrylic acid esters having an aromatic ring, such as benzyl (meth)acrylate; and (meth)acrylic acid esters having a heterocyclic ring, such as tetrahydrofurfuryl (meth)acrylate.

The hydrogel may or may not contain acrylic acid as an acrylic monomer. The presence of an appropriate amount of acrylic acid in the hydrogel can increase the hardness of the hydrogel while maintaining the inherent adhesive force of the hydrogel. However, since an odor is generated in the hydrogel by containing acrylic acid, the amount of acrylic acid in the hydrogel is preferably smaller. The amount of acrylic acid is, for example, 5 mass% or less, and preferably 1 mass% or less, based on the hydrogel. More preferably, acrylic acid is not added to the hydrogel.

The amount of the acrylic monomer added is preferably within the range of 98.5 mass% to 99.97 mass% based on the total amount of the polymer matrix, in terms of formation of the hydrogel, resistance to tearing, and hardness. Similarly, the content of the structural unit derived from the acrylic monomer in the polymer matrix is preferably within the range of 98.5 mass% to 99.97 mass%.

The amount of the acrylic monomer added is more preferably 99.5 mass% to 99.85 mass% based on the total amount of the polymer matrix, in terms of formation of the hydrogel, resistance to tearing, and hardness. Similarly, the content of the structural unit derived from the acrylic monomer in the polymer matrix is more preferably within the range of 99.5 mass% to 99.85 mass%.

Further, the amount of the acrylic monomer added is preferably within the range of 2 mass% to 60 mass% based on the hydrogel, in terms of formation of the hydrogel, resistance to tearing, and hardness. The content of the acrylic monomer in the hydrogel is preferably 2 mass% or more. In terms of allowing the movement of ions in the hydrogel, the content of the acrylic monomer in the hydrogel is preferably 60 mass% or less. The content of the polymer matrix in the hydrogel is preferably within the range of 5 mass% to 50 mass%, more preferably within the range of 10 mass% to 40 mass%, and even more preferably within the range of 13 mass% to 35 mass%. The content of the structural unit derived from the crosslinkable monomer in the hydrogel is preferably within the range of 0.01 mass% to 0.1 mass%, and more preferably within the range of 0.03 mass% to 0.05 mass%.

As the crosslinkable monomer, it is preferable to use a monomer that contains two or more polymerizable carbon-carbon double bonds in the molecule. Specific examples include polyfunctional (meth)acrylamides and polyfunctional (meth)acrylic acid esters, such as methylenebis(meth)acrylamide, ethylenebis(meth)acrylamide, (poly)ethylene glycol di(meth)acrylate, (poly)propylene glycol di(meth)acrylate, glycerol di(meth)acrylate, and glycerol tri(meth)acrylate; tetraallyloxyethane; diallyl ammonium chloride; and the like. These may be used singly or in a combination of two or more. As the crosslinkable monomer containing two or more polymerizable carbon-carbon double bonds in the molecule, a polyglycerol derivative that is a polyfunctional compound containing two or more (meth)acryloyl groups or vinyl groups and having a molecular weight of 400 or more, as described in JP2803886B, can also be used. The above polyfunctional (meth)acrylamides, polyfunctional (meth)acrylic acid esters, and polyglycerol derivative are included in acrylic monomers.

The amount of the crosslinkable monomer added is preferably within the range of 0.02 mass% to 1.5 mass% based on the total amount of the polymer matrix, in terms of adhesive force, adhesion in repeated use, resistance to tearing, and hardness. When the amount of the crosslinkable monomer added is 0.02 mass% or more, the crosslinking density is maintained, and good shape stability is obtained; at the same time, the cohesive force of the gel can be increased, and the adhesive force can be sufficiently high. Further, when being peeled off, the sheet of the resulting gel can be smoothly peeled off from the adherend, is less likely to tear, and is easy to handle. When the amount of crosslinkable monomer added is 1.5 mass% or less, the adhesive force and/or adhesion in repeated use can be maintained at a high level, and a flexible gel can be obtained. Similarly, the content of the structural unit derived from the crosslinkable monomer in the polymer matrix is preferably within the range of 0.02 mass% to 1.5 mass%.

Further, the amount of the crosslinkable monomer added is preferably within the range of 0.01 mass% to 0.1 mass%, and more preferably within the range of 0.03 mass% to 0.05 mass%, based on the hydrogel, in terms of, for example, the adhesive force, adhesion in repeated use, and handling of the gel sheet. Similarly, the content of the structural unit derived from the crosslinkable monomer in the hydrogel is preferably within the range of 0.01 mass% to 0.1 mass%, and more preferably within the range of 0.03 mass% to 0.05 mass%.

In terms of adhesion in repeated use, resistance to tearing, and hardness, it is preferred that the amount of the acrylic monomer added as a constituent monomer of the polymer matrix is within the range of 98.5 mass% to 99.97 mass% based on the total amount of the polymer matrix and that the amount of the crosslinkable monomer added as a constituent monomer of the polymer matrix is within the range of 0.03 mass% to 1.5 mass% based on the total amount of the polymer matrix. It is more preferred that the amount of the acrylic monomer added is within the range of 99.5 mass% to 99.85 mass% based on the total amount of the polymer matrix and that the amount of the crosslinkable monomer added is within the range of 0.15 mass% to 0.5 mass% based on the total amount of the polymer matrix.

In terms of adhesion in repeated use, resistance to tearing, and hardness, it is preferred that the content of the structural unit derived from the acrylic monomer in the polymer matrix is within the range of 98.5 mass% to 99.97 mass% and that the content of the structural unit derived from the crosslinkable monomer in the polymer matrix is within the range of 0.03 mass% to 1.5 mass%. It is more preferred that the content of the structural unit derived from the acrylic monomer in the polymer matrix is within the range of 99.5 mass% to 99.85 mass% and that the content of the structural unit derived from the crosslinkable monomer in the polymer matrix is within the range of 0.15 mass% to 0.5 mass%.

It is preferred that the copolymer of the acrylic monomer and the crosslinkable monomer does not contain an additional monomer that constitutes the copolymer, other than the acrylic monomer and the crosslinkable monomer.

However, the copolymer may contain additional monomers.

The water-soluble polymer is added in order to impart to the hydrogel adhesive force to an adherend, adhesion in repeated use, and gel cohesive force (hardness).

The water-soluble polymer is preferably a nonionic water-soluble polymer having no ionic group in the molecule.

The water-soluble polymer is, for example, one or more members selected from the group consisting of poly N-vinyl pyrrolidone (PVP), polyethylene oxide (PEO), polyvinyl alcohol (PVA), and partially saponified polyvinyl alcohol. The water-soluble polymer is preferably PVP.

The content of the water-soluble polymer in the hydrogel is not particularly limited, and is preferably within the range of 0.1 mass% to 13 mass%, more preferably within the range of 0.5 mass% to 12 mass%, and even more preferably within the range of 0.5 mass% to 10 mass%, based on the hydrogel.

The content of the water in the hydrogel is not particularly limited, and is preferably 10 to 60 mass%, more preferably 10 to 45 mass%, and even more preferably 15 to 30 mass%, based on the hydrogel. If the content of the water is too low, the water content of the hydrogel relative to the equilibrium water content becomes low, and the hydrogel becomes highly hygroscopic, which may cause the hydrogel to deteriorate (e.g., swell) over time. If the content of the water is too high, the water content of the hydrogel relative to the equilibrium water content becomes high, which may cause shrinkage and changes in physical properties of the hydrogel due to dryness.

The polyhydric alcohol is added in order to impart wettability to the hydrogel. The polyhydric alcohol is not particularly limited. Examples include diols, such as ethylene glycol, triethylene glycol, 1,6-hexanediol, 1,9-nonanediol, propylene glycol, and butanediol; polyhydric alcohols that are trihydric or higher alcohols, such as glycerol, pentaerythritol, and sorbitol; polyhydric alcohol condensates, such as polyethylene glycol, polypropylene glycol, and polyglycerol; modified polyhydric alcohols, such as polyoxyethylene glycerol; and the like.

Of these polyhydric alcohols, it is preferable to use a polyhydric alcohol that is liquid in a temperature range in which the hydrogel is used (for example, about 20°C when the hydrogel is used indoors). Specifically, for example, one or more members selected from the group consisting of ethylene glycol, triethylene glycol, propylene glycol, polypropylene glycol, polyethylene glycol, polyglycerol, and glycerol are preferred.

The content of the polyhydric alcohol in the hydrogel is not particularly limited, and is preferably within the range of 20 to 70 mass%, and more preferably within the range of 25 to 65 mass%, based on the hydrogel. The content of the polyhydric alcohol is preferably higher than the content of the water; however, the content of the polyhydric alcohol may be less than or equal to the content of the water. If the content of the polyhydric alcohol is too low, the resulting hydrogel is poor in moisture retention and plasticity, and water evaporation becomes significant, resulting in a lack of stability of the hydrogel over time, as well as a lack of flexibility, which may lead to insufficient adhesion. If the content of the polyhydric alcohol is too high, it exceeds the amount of the polyhydric alcohol that the polymer matrix can retain, and the physical properties of the hydrogel change due to bleeding out of the polyhydric alcohol from the surface of the hydrogel, which may lead to insufficient adhesion. Thus, the content of the polyhydric alcohol is suitably determined in view of balancing these.

If necessary, the hydrogel of the present invention may also contain an electrolyte, which imparts conductivity to the hydrogel.

The electrolyte is not particularly limited. Examples include alkali metal halides, such as sodium halide, lithium halide, and potassium halide; alkaline earth metal halides, such as magnesium halide and calcium halide; other metal halides; and the like. As the electrolyte, hypochlorites, chlorites, chlorates, perchlorates, hydrochlorides, sulfates, carbonates, nitrates, and phosphates of various metals can also be preferably used. As the electrolyte, inorganic salts, such as ammonium salts and complex salts; salts of monovalent organic carboxylic acids, such as acetic acid, benzoic acid, and lactic acid; salts of polyvalent organic carboxylic acids, such as tartaric acid; monovalent, divalent, or higher valent salts of polyvalent carboxylic acids, such as phthalic acid, succinic acid, adipic acid, and citric acid; metal salts of organic acids, such as sulfonic acids and amino acids; organic ammonium salts; and the like are also preferable.

The content of the electrolyte in the hydrogel is preferably 0.05 to 10 mass%, and more preferably 0.1 to 6 mass%, based on the hydrogel, to impart conductivity to the hydrogel. If the content of the electrolyte is too low, the impedance becomes high, and thus, the conductivity becomes worse. As the content of the electrolyte increases, the impedance decreases; however, if the content of the electrolyte is too high, the impedance no longer decreases, which is not preferable in terms of cost.

Moreover, a base, such as sodium hydroxide, may be suitably added to the hydrogel in order to adjust the pH.

Furthermore, the hydrogel according to this embodiment may contain an amphiphilic polymer, such as polyacrylic acid or a salt thereof, as necessary, in order to enhance the adhesive force.

Examples of amphiphilic polymers include a copolymer of acrylic acid and methacrylic acid; a polymer containing N-alkyl sulfonic acid acrylamide in a structural unit; and the like. These may be used singly or in a combination of two or more.

In the copolymer of acrylic acid and methacrylic acid, the copolymerization ratio (molar ratio) of acrylic acid and methacrylic acid is preferably 9:1 to 1:9.

If the content of the copolymer of acrylic acid and methacrylic acid is too low, it is difficult to obtain the desired adhesive force. On the other hand, if the content of the copolymer of acrylic acid and methacrylic acid is too high, the hydrogel become hard, resulting in a decrease in adhesive force. Thus, the content of the copolymer of acrylic acid and methacrylic acid is suitably determined, taking into consideration the balance between these. Specifically, the content of the copolymer of acrylic acid and methacrylic acid is preferably 0.03 to 3 mass%, and more preferably 0.2 to 2 mass%, based on the hydrogel.

The copolymer of acrylic acid and methacrylic acid can be produced by a method such as radical polymerization, redox reaction, or light irradiation. Examples of usable copolymers of acrylic acid and methacrylic acid include commercially available products such as Jurymer AC-20H and AC-20L (trade names) produced by Toagosei Co., Ltd. and FL-200 (trade name) produced by Nippon Shokubai Co., Ltd.

The weight average molecular weight of the polymer containing N-alkyl sulfonic acid acrylamide in a structural unit is not particularly limited, and is preferably 7000000 or less in order to easily prepare a mixture liquid and allow the resulting hydrogel to exhibit optimal adhesive force. The weight average molecular weight of the polymer containing N-alkyl sulfonic acid acrylamide in a structural unit is also preferably 500000 or more in order to obtain a cohesive gel.

The content of the polymer containing N-alkyl sulfonic acid acrylamide in a structural unit is preferably 0.1 to 40 mass%, and more preferably 0.4 to 15 mass%, based on the hydrogel.

The polymer containing N-alkyl sulfonic acid acrylamide in a structural unit may be a copolymer with another polymer. Examples of the copolymer that is commercially available include a copolymer of acrylic acid and N-alkyl sulfonic acid acrylamide. Specifically, a copolymer of acrylic acid and acrylamidomethyl propane sulfonic acid (Aronvis AH-305 (trade name) produced by Toagosei Co., Ltd.) or the like can be used.

When the polymer containing N-alkyl sulfonic acid acrylamide in a structural unit is a copolymer with another polymer, the copolymerization ratio (molar ratio) of the polymer containing N-alkyl sulfonic acid acrylamide to the other polymer is preferably 2:8 to 8:2, and more preferably 2:8 to 5:5.

The hydrogel may contain other additives, if necessary. Examples of the other additives include rust inhibitors, fungicides, antioxidant, antifoaming agents, stabilizers, surfactants, colorants, and the like.

The hydrogel of the present invention has a tensile strength of 0.5 N/20 mm to 3.0 N/20 mm. In the present specification, the tensile strength refers to the tensile strength [N/20 mm] when a hydrogel cut to a size of 120 mm $\times$ 20 mm $\times$ 0.4 mm as a test specimen is pulled at a tensile rate of 20 mm/min under an environment of a temperature of $23\pm5°C$ and a humidity of $55\%\pm10\%$, with a tensile tester (Tensilon (registered trademark) universal testing instrument RTE-1210 produced by Orientec Co., Ltd.). If the tensile strength of the hydrogel is less than 0.5 N/20 mm, the hydrogel may tear during peeling. If the tensile strength of the hydrogel exceeds 3.0 N/20 mm, the strength of the gel increases; however, the adhesion may decrease.

Method for Producing Hydrogel

The hydrogel can be obtained by uniformly dispersing, in water, the above-mentioned materials other than water, and a polymerization initiator and polymerizing (crosslinking) the dispersion by, for example, heating or ultraviolet irradiation. Dispersing includes not only a state in which a solute is dispersed in water without mixing with water, but also dissolution in which a solute is mixed with water to form a homogeneous phase mixture.

The polymerization initiator may be a thermal polymerization initiator or a photopolymerization initiator. Known thermal polymerization initiators or photopolymerization initiators for polymerizing acrylic monomers can be used. The content of the polymerization initiator is not particularly limited, and is preferably 0.01 mass% or more and 1 mass% or less, based on the dispersion (which is a composition before polymerization and may be referred to as a "monomer mixture liquid") excluding the polymerization initiator. Further, when polymerization is performed by ultraviolet irradiation, the integrated amount of ultraviolet irradiation varies depending on, for example, the content of the polymerization initiator. For example, the integrated amount of ultraviolet irradiation is preferably within the range of 800 mJ/cm$^2$ to 10000 mJ/cm$^2$, and more preferably within the range of 2000 mJ/cm$^2$ to 10000 mJ/cm$^2$.

By setting the amount of the polymerization initiator and the amount of ultraviolet irradiation appropriately, the reaction rate of the acrylic monomer can be suitably adjusted to be, for example, 99.8% or more. Although the amount of ultraviolet irradiation is not particularly limited, it is preferable to perform irradiation with ultraviolet light having a peak intensity of 70 to 150 mW/cm$^2$. When the peak intensity is 70 mW/cm$^2$ or more, low-molecular-weight components are contained in a large amount in the hydrogel, resulting in improved adhesive function. When the peak intensity is 150 mW/cm$^2$ or less, components having appropriate molecular weights are easily obtained.

The hydrogel can be formed into a desired shape, such as a sheet, by pouring the monomer mixture liquid into a container having a desired shape, such as a bottomed container having a substantially rectangular cross-section and performing polymerization by, for example, ultraviolet irradiation. The hydrogel formed into a sheet may have any shape according to the purpose. For example, the sheet of the hydrogel may have a substantially rectangular shape or a substantially circular shape; however, the shape of the sheet of the hydrogel is not limited thereto. Hereinafter, the hydrogel in the form of a sheet is referred to as a "hydrogel sheet" or simply a "gel sheet."

Further Configuration of Hydrogel and Gel Sheet

In the hydrogel of the present invention, it is preferred that an intermediate substrate is embedded along the in-plane direction. The in-plane direction of the hydrogel refers to any direction in the plane perpendicular to the thickness direction of the hydrogel. The presence of the intermediate substrate leads to reinforcement of the hydrogel, improvement in shape retention at the time of cutting, and the like. In a specific embodiment, the intermediate substrate may be composed of a nonwoven fabric or a woven fabric. Examples of materials of the nonwoven fabric and the woven fabric include natural fibers such as cellulose, silk, and hemp, synthetic fibers such as polyester, nylon, rayon, polyethylene, polypropylene, and polyurethane, and blends thereof. A binder may be used as necessary. Further, these materials may be colored as necessary.

Examples of the method for producing the nonwoven fabric include, but are not limited to, a dry method, a wet method, a spunbonding method, a meltblown method, an air-laid method, a chemical bonding method, a thermal bonding method, a needle punching method, and a spunlacing method. To control the position of the intermediate substrate, it is preferable to use a production method depending on the basis weight and the material so as to have an even basis weight. The woven fabric is also not particularly limited, and examples include a plain weave, tricot, raschel, and the like. The woven fabric can be appropriately selected.

The basis weight of the woven fabric or the nonwoven fabric is not particularly limited as long as it is the basis weight that enables the fabric to have predetermined physical properties as an intermediate substrate. For example, the basis weight of the woven fabric or the nonwoven fabric is preferably 10 to 40 $g/m^2$, and more preferably 10 to 28 $g/m^2$. If the basis weight of the woven fabric or the nonwoven fabric is too small, it may be impossible to reinforce the gel sheet, or the unevenness of the basis weight may increase. As a result, the permeability of a liquid varies depending on the location during the production of the gel sheet, which may change the position of the intermediate substrate. If the basis weight is too large, the intermediate substrate becomes hard, which may impair the followability of the hydrogel to the skin and adversely affect the conductivity. The basis weight is thus suitably determined, taking into consideration the balance between these.

If the intermediate substrate is too thick, the permeability of a liquid decreases, which may adversely affect the conductivity. On the other hand, if the intermediate substrate is too thin, it may be impossible to reinforce the gel sheet, or the position of the intermediate substrate may change, as in the case in which the basis weight is too small. The thickness of the intermediate substrate is thus suitably determined, taking these into consideration. The thickness of the intermediate substrate is preferably within the range of 0.05 mm to 2.0 mm, more preferably 0.05 mm to 0.5 mm, and particularly preferably 0.08 mm to 0.3 mm.

If the hydrogel of the present invention is too thick, the shear stress decreases. If the hydrogel of the present invention is too thin, the cohesive force decrease. The thickness of the hydrogel of the present invention is suitably selected, taking these into consideration. The thickness of the hydrogel of the present invention is preferably within the range of 0.2 mm to 2.0 mm, more preferably 0.3 mm to 1.5 mm, and even more preferably 0.5 mm to 1.5 mm.

The storage modulus (at a frequency of 0.1 Hz) of the hydrogel of the present invention is not particularly limited. A preferred lower limit is 2000 Pa, and a preferred upper limit is 9000 Pa. If the storage modulus is less than the lower limit, the gel becomes too soft and thus becomes likely to tear, and there are concerns that the handling and the shape stability become poor and that the processability in processing into a specific shape decreases. If the storage modulus exceeds the upper limit, the gel becomes too hard, and the adhesive force may decrease too much, although the handling and the processability can be improved. The lower limit of the storage modulus of the hydrogel of the present invention is more preferably 3000 Pa, and even more preferably 4000 Pa. The upper limit of the storage modulus of the hydrogel is more preferably 8000 Pa, and even more preferably 7000 Pa.

The adhesive force of the hydrogel of the present invention to a Bakelite plate is not particularly limited. A preferred lower limit is 1 N/20 mm, and a preferred upper limit is 15 N/20 mm. If the adhesive force is less than the lower limit, the adhesive force to the skin is insufficient, and the gel may fall off the element when it is used for an electrode. If the adhesive force exceeds the upper limit, the adhesive force to the skin is too strong, which may cause pain or redness when the gel is peeled off. The lower limit of the adhesive force of the hydrogel of the present invention to a Bakelite plate is more preferably 2 N/20 mm, and even more preferably 3 N/20 mm. The upper limit of the adhesive force of the hydrogel of the present invention to a Bakelite plate is more preferably 9 N/20 mm, and even more preferably 7 N/20 mm.

The process for producing the hydrogel sheet comprising an intermediate substrate is not particularly limited. The detailed conditions vary depending on, for example, the composition of the gel material, the material of the intermediate substrate, and the thickness. For example, a method such as the following can be suitably used: a method in which an intermediate substrate is held in the air with at least a certain degree of tension applied so that the deformation of the intermediate substrate in the vertical direction is minimized, and a monomer mixture liquid is poured onto the upper and lower sides of the intermediate substrate and polymerized by light irradiation or the like to form a sheet; a method in which two sheet-shaped gel materials with a smooth surface are prepared, and an intermediate substrate held with at least a certain degree of tension applied is sandwiched between these gel materials to form a composite; or a method in which a sheet-shaped gel material with a smooth surface is prepared, an intermediate substrate is placed on the gel material while at least a certain degree of tension is applied, and a monomer mixture liquid is poured on the intermediate substrate and further polymerized by light irradiation or the like. When the production process is a continuous process, the intermediate substrate and the gel material may be formed into rolls, removed therefrom, and then suitably cut into sheets.

Fig. 1 (A) is a schematic plan view of an embodiment of the gel sheet, and Fig. 1 (B) is a cross-sectional view of the gel sheet of Fig. 1 (A) taken along the line 1B-1B in Fig. 1 (A). The gel sheet 1 comprises a gel material 10 formed from the hydrogel of the present invention, and an intermediate substrate 12 embedded in the gel material 10. The details of the intermediate substrate 12 are as described above. In this embodiment, a base film 14 is provided on one side of the gel sheet 1, and a top film 16 is provided on the side opposite to the side on which the base film 14 is provided. However, the base film 14 and the top film 16 may be omitted.

For example, a resin film made of a resin such as polyester, polyolefin, polystyrene, or polyurethane, paper, or paper on which the above resin film is laminated may be used as the base film 14.

It is preferred that the surface of the base film 14 that comes into contact with the gel sheet 1 is subjected to a release treatment. The release treatment method is, for example, silicone coating, and particularly preferably bake-type silicone coating in which crosslinking and curing are performed by using heat or ultraviolet light. The film to be subjected to a release treatment is particularly preferably a biaxially oriented polyethylene terephthalate (PET) film, an oriented polypropylene (OPP) film, or the like.

The same material as that of the base film can be basically used for the top film 16. However, when polymerization is performed in a state in which the top film is provided by, for example, applying ultraviolet light from above the top film, it is preferable to select a film made of a material that does not block light so as not to interfere with photopolymerization.

The hydrogel of the present invention is excellent in flexibility, water retention, and the like and thus can be used in a wide range of fields such as medical care, cosmetics, food, chemistry, civil engineering, agriculture, bioengineering, and sports. For example, the hydrogel of the present invention can be used as a medical electrode hydrogel, a cooling gel, a cosmetic face mask, a cell culture medium, or the like. Preferably, the hydrogel of the present invention can be used as a medical electrode hydrogel to be placed between an electrode comprising a conductive material and a skin surface.

The present invention may also include the following.

1. A hydrogel comprising:

a polymer matrix formed from a copolymer of an acrylic monomer and a crosslinkable monomer;
a water-soluble polymer;
water; and
a polyhydric alcohol,
the hydrogel having a tensile strength of 0.5 to 3.0 N/20 mm.

2. The hydrogel according to Item 1, wherein the water-soluble polymer is polyvinylpyrrolidone.

3. The hydrogel according to Item 1 or 2, wherein the acrylic monomer is an acrylamide-based monomer.

4. The hydrogel according to any one of Items 1 to 3, wherein the content of a structural unit derived from the acrylic monomer is within the range of 98.5 mass% to 99.97 mass% based on the total amount of the polymer matrix.

5. The hydrogel according to any one of Items 1 to 4, wherein the content of a structural unit derived from the crosslinkable monomer is 0.02 mass% to 1.5 mass% based on the total amount of the polymer matrix.

6. The hydrogel according to any one of Items 1 to 5, wherein the content of a structural unit derived from the crosslinkable monomer in the hydrogel is 0.01 mass% to 0.1 mass%.

7. The hydrogel according to any one of Items 1 to 6, wherein the concentration of acrylic acid in the hydrogel is 5 mass% or less.

8. The hydrogel according to any one of Items 1 to 7, wherein the concentration of acrylic acid in the hydrogel is 1 mass% or less.

9. The hydrogel according to any one of Items 1 to 8, wherein the water-soluble polymer comprises one or more members selected from the group consisting of poly N-vinyl pyrrolidone, polyethylene oxide, polyvinyl alcohol, and partially saponified polyvinyl alcohol.

10. The hydrogel according to any one of Items 1 to 9, wherein the content of the water-soluble polymer is 0.1 mass% to 13 mass%.

11. The hydrogel according to any one of Items 1 to 10, wherein the content of the water-soluble polymer is 0.5 mass% to 10 mass%.

12. The hydrogel according to any one of Items 1 to 11, wherein the content of the water is 10 mass% to 60 mass%.

13. The hydrogel according to any one of Items 1 to 12, wherein the polyhydric alcohol comprises one or more members selected from the group consisting of ethylene glycol, triethylene glycol, propylene glycol, polypropylene glycol, polyethylene glycol, polyglycerol, and glycerol.

14. The hydrogel according to any one of Items 1 to 13, wherein the content of the polyhydric alcohol is 20 mass% to 70 mass%.

15. The hydrogel according to any one of Items 1 to 14, further comprising an electrolyte.

16. The hydrogel according to any one of Items 1 to 15, wherein the electrolyte comprises one or more members selected from the group consisting of alkali metal halides, alkaline earth metal halides, metal hypochlorites, metal chlorites, metal chlorates, metal perchlorates, metal hydrochlorides, metal sulfates, metal carbonates, metal nitrates, metal phosphates, ammonium salts, complex salts, salts of monovalent organic carboxylic acids, monovalent, divalent, and higher valent salts of tartaric acid, phthalic acid, succinic acid, adipic acid, and citric acid, metal salts of sulfonic acids and amino acids, and organic ammonium salts.

17. The hydrogel according to Item 15 or 16, wherein the content of the electrolyte is 0.05 mass% to 6 mass%.

18. The hydrogel according to any one of Items 1 to 17, wherein the content of the polymer matrix in the hydrogel is 5 mass% to 50 mass%.

19. The hydrogel according to any one of Items 1 to 18, wherein an intermediate substrate is embedded along an in-plane direction.

20. The hydrogel according to Item 19, wherein the intermediate substrate is a nonwoven fabric.

21. The hydrogel according to any one of Items 1 to 20, which has a thickness of 0.5 to 1.5 mm.

22. The hydrogel according to any one of Items 1 to 21, which has a storage modulus of 2000 to 9000 Pa.

23. A medical electrode hydrogel to be placed between an electrode comprising a conductive material and a skin surface, the medical electrode hydrogel comprising the hydrogel according to any one of Items 1 to 22.

24. A method for producing the hydrogel according to any one of Items 1 to 22, comprising irradiating a polymerizable solution with ultraviolet light having a peak intensity of 70 to 150 mW/cm$^2$, the polymerizable solution comprising an acrylic monomer, a crosslinkable monomer, a water-soluble polymer, water, and a polyhydric alcohol and having a crosslinkable monomer content of 0.03 to 0.05 mass%.

Examples

The present invention is described in more detail below with reference to Examples and Comparative Examples; however, the present invention is not limited to these Examples.

1. Preparation of Hydrogel

Preparation of Hydrogel of Example 1

(1) Preparation of Monomer Mixture Liquid

As shown in Table 1, 20 mass% of acrylamide as an non-crosslinkable monomer, 0.04 mass% of methylenebisacrylamide as a crosslinkable monomer, 0.5 mass% of polyvinylpyrrolidone (Creejus K-90 produced by DKS Co. Ltd.) as a water-soluble polymer, and 18 mass% of ion-exchanged water were mixed, stirred, and uniformly dissolved in a stirring and mixing container. Thereafter, 58 mass% of glycerol as a moisturizing agent was added, and the mixture was stirred until uniform. Subsequently, 2.5 mass% of sodium chloride as an electrolyte and 0.96 mass% in total of citric acid, sodium benzoate, a photopolymerization initiator, and a surfactant as other additives were added, and the mixture was stirred until complete dissolution. The mixture was stirred until uniform to obtain a transparent monomer mixture liquid.

(2) Production of Hydrogel

Example 1

The obtained mixture liquid was dropped on a base film coated with silicone and allowed to pass through a certain clearance to spread the liquid uniformly. An intermediate substrate (nonwoven fabric) was placed thereon, and the mixture liquid was further dropped on the intermediate substrate. A top film coated with silicone was placed thereon to spread the liquid uniformly, followed by fixing so that the thickness was 0.75 mm. Ultraviolet irradiation was performed at a peak illuminance of 130 mW/cm$^2$ in an energy amount of 3000 mJ/cm$^2$ using a metal halide lamp, thereby obtaining a hydrogel sheet of Example 1 having a thickness of 0.75 mm.

Examples 2 to 8

Hydrogels were produced using mixture liquids in the same manner as in Example 1, except that the mass% of each component and the gel thickness were as shown in Table 1. In Example 5, 0.5 mass% of an acrylic acid-acrylamidomethyl propane sulfonic acid copolymer (Aronvis AH-305X produced by Toagosei Co., Ltd.) was added as an amphiphilic polymer. In Example 8, the thickness of the gel was 0.9 mm.

Comparative Example 1

As shown in Table 1, 14.4 mass% of acrylic acid and 9.6 mass% of tert-butyl acrylamide sulfonic acid (TBAS) as non-crosslinkable monomers, 0.04 mass% of methylenebisacrylamide as a crosslinkable monomer, and 3 mass% of polyvinylpyrrolidone (Creejus K-90 produced by DKS Co. Ltd.) as a water-soluble polymer were mixed in a stirring and mixing container. Further, 8.0 mass% of a 50 mass% NaOH solution was added to adjust the pH to 4 to 5, and 17.3 mass% of ion-exchanged water was added, followed by mixing. Thereafter, 44 mass% of glycerol as a moisturizing agent was added, and the mixture was stirred until uniform. Subsequently, 0.5 mass% of sodium chloride as an electrolyte and 3.16 mass% in total of citric acid, sodium benzoate, a photopolymerization initiator, a surfactant, and N-vinyl-2-caprolactam as other additives were added, and the mixture was stirred until complete dissolution. The mixture was stirred until uniform to obtain a transparent mixture liquid. A hydrogel of Comparative Example 1 was produced using the obtained mixture liquid by the same production method as that in Example 1.

Comparative Example 2

A mixture liquid was prepared in the same manner as in Example 1, except that the mass% of each component was as shown in Table 1. A hydrogel was produced using this mixture liquid in the same manner as in Example 1, except that infrared irradiation was performed at a peak illuminance of 50 mW/cm$^2$ in an energy amount of 3000 mJ/cm$^2$ using a metal halide lamp.

Comparative Examples 3 to 5

Mixture liquids were prepared in the same manner as in Example 1, except that the mass% of each component was as shown in Table 1. Hydrogels were produced using these mixture liquids in the same manner as in Example 1.
In Example 5, 1 mass% of an acrylic acid-acrylamidomethyl propane sulfonic acid copolymer (Aronvis AH-305X produced by Toagosei Co., Ltd.) was added as an amphiphilic polymer.

2. Evaluation Methods

The obtained hydrogels of Examples 1 to 8 and Comparative Examples 1 to 5 were evaluated for the following items.

(1) Evaluation of Impedance

Each hydrogel was individually cut to a size of 20 mm × 20 mm, and the top film was peeled off. The exposed gel surface (layer A) was attached to stainless steel SUS304. Another sample was prepared in the same manner. The base film in each sample was peeled off, and the exposed gel surfaces (layers B) were bonded to each other to obtain a test specimen. The voltage applied between the SUS plates was read with an oscilloscope (produced by Tektronix; TDS3014) when a current was applied to the test specimen under the conditions of input voltage: 10 V, resistance: 1 MΩ, and frequency: 1 kHz and 10 Hz, and the electrode impedance value was determined by the following formula (1) (Ohm's law).

$$|Z| = E/I \quad (1)$$

wherein |Z| is the impedance value (Ω) of an electrode, E is a voltage value (V) read with an oscilloscope, and I is a current applied at 10 (μA) to the electrode.

(2) Solubility of Mixture Liquid

The appearance of the mixture liquids prepared for the production of the hydrogels of the Examples and Comparative Examples shown in Table 1 was visually evaluated. A case in which complete dissolution was observed was evaluated as Yes, and a case in which even a portion of insoluble matter remained was evaluated as No.

(3) Evaluation of Adhesive Force

Evaluation of Adhesive Force to Bakelite Plate

Each hydrogel was individually cut to a size of 120 mm × 20 mm, and the base film was peeled off. The exposed gel surface was attached to a Bakelite plate, followed by pressing by reciprocating a 2-kg press roller once, thereby obtaining a test specimen. Measurement was performed with a rheometer (CR-500DX produced by Sun Scientific Co., Ltd.) under the conditions of angle: 90 degrees and speed: 300 mm/min, in accordance with JIS-Z0237 2009. Stress values (N/20 mm) were measured at predetermined peeling-off points in time (30, 40, 50, 60, and 70 mm) from the measurement starting point, and the average was calculated from the values in three tests (a total of 15 points) and regarded as the adhesive force of the hydrogel. The measurement was performed under an environment of a temperature of 23±5°C and a humidity of 55%±10%.

Evaluation of Initial Adhesive Force to Skin

Each hydrogel was individually cut to a size of 120 mm × 20 mm, and the top film was peeled off. Synthetic paper coated with an inorganic filler was attached to the exposed gel surface to create a handle used at the time of measurement, thereby obtaining a test specimen. Thereafter, five subjects (men and women in their 20s to 50s) were brought into a measurement room under an environment of a temperature of 23±5°C and a humidity of 55%±10% 15 minutes before application of the test specimen. The test specimen was applied to an inner forearm portion of each subject with as little body hair as possible and maintained in that state for 30 minutes. Measurement was performed with a rheometer (CR-500DX produced by Sun Scientific Co., Ltd.) under the conditions of angle: 180 degrees and speed: 1000 mm/min. Stress values (N/20 mm) were measured at predetermined peeling-off points in time (30, 40, 50, 60, and 70 mm) from the measurement starting point, and the average was calculated from the values in two tests (a total of 10 points) and regarded as the initial adhesive force (I) of the hydrogel to the skin.

Evaluation of Adhesive Force to Skin after 20 Applications

The same test specimens as those used for evaluation of the initial adhesive force to the skin were applied to the inner forearm of five subjects, and peeling off and application were repeated 20 times at 1-minute intervals. Stress values (N/20 mm) at points in time (30, 40, 50, 60, and 70 mm) of the 20th peeling from the measurement starting point were measured with a rheometer (CR-500DX produced by Sun Scientific Co., Ltd.). The average was calculated from the values in two tests (a total of 10 points) and regarded as the adhesive force (II) of the hydrogel to the skin after 20 applications.

As an index of adhesion in repeated use, the ratio of the adhesive force to the skin after 20 applications to the initial adhesive force to the skin (i.e., the (II)/(I) ratio) was calculated. A (II)/(I) ratio of 0.8 or more was evaluated as A, a (II)/(I) ratio of 0.7 or more and less than 0.8 was evaluated as B, and a (II)/(I) ratio of less than 0.7 was evaluated as C.

(4) Evaluation of Odor

A trained panel sniffed the surface of each of the hydrogels of the Examples and Comparative Examples. A case in which the panel did not sense odor was evaluated as No, and a case in which the panel sensed odor was evaluated as Yes.

(5) Evaluation of Gel Tearing

Each of the hydrogels of the Examples and Comparative Examples cut to a size of 50 mm × 50 mm was individually applied to the inner arm of a trained panel and peeled off 3 minutes after the application. A case in which no gel remained on the skin was evaluated as Pass, and a case in which even a portion of the gel remained was evaluated as Fail.

(6) Evaluation of Tensile Strength

Each hydrogel was individually cut to a size of 120 mm × 20 mm × 0.4 mm to prepare a test specimen. The tensile strength [N/20 mm] when the test specimen was pulled at a tensile rate of 20 mm/min was measured under an environment of a temperature of $23\pm5°C$ and a humidity of $55\%\pm10\%$ with a tensile tester (Tensilon (registered trademark) universal testing instrument RTE-1210 produced by Orientec Co., Ltd.).

(7) Evaluation of Storage Modulus

The storage modulus of each hydrogel was measured. Specifically, viscoelasticity measurement was performed at 1% strain, 23°C, and a frequency of 0.1 Hz, with a viscoelasticity measuring device (MR-102 produced by Anton Paar GmbH). A 25φ gel piece was attached to a 25φ SUS parallel plate. A jig was pressed against the gel piece to a load point of 1N, and the storage modulus at 0.1 Hz was then calculated. The higher the storage modulus, the more elastic the gel is, which serves as an index of hardness.

3. Results

Table 1 shows the results. When the hydrogel of Comparative Example 4 was produced, the viscosity of the mixture liquid was adjusted to be too high, and the water-soluble polymer aggregated, making uniform dissolution difficult; as a result, a homogeneous gel could not be obtained in Comparative Example 4. The tensile strength of the hydrogels of Examples 1 to 8 was higher than that of the hydrogels of Comparative Examples 1 to 3 and 5, indicating that the hydrogels of Examples 1 to 8 had sufficient hardness but were resistant to tearing. The hydrogels of Examples 1 to 8 were also excellent in adhesion in repeated use. Further, since the hydrogels of Examples 1 to 8 contained no acrylic acid as a monomer, no odor was generated. It was confirmed that the hydrogels of Comparative Examples 1 to 5 were torn and were poor in adhesion in repeated use.

The above results show that the hydrogels of the Examples were excellent in adhesion in repeated use and were resistant to tearing.

Table 1

| Proportions of components (mass%) | | | Example | | | | | | | | Comparative Example | | | | |
|---|---|---|---|---|---|---|---|---|---|---|---|---|---|---|---|
| | | | 1 | 2 | 3 | 4 | 5 | 6 | 7 | 8 | 1 | 2 | 3 | 4 | 5 |
| | Monomer-derived component | Non-crosslinkable monomer (acrylamide) | 20 | 20 | 20 | 20 | 18 | 19 | 19 | 20 | - | 20 | 20 | 20 | 19 |
| | | Non-crosslinkable monomer (acrylic acid) | - | - | - | - | - | - | - | - | 14.4 | - | - | - | - |
| | | Non-crosslinkable monomer (TBAS) | - | - | - | - | - | - | - | - | 9.6 | - | - | - | - |
| | | Crosslinkable monomer | 0.040 | 0.040 | 0.040 | 0.040 | 0.040 | 0.030 | 0.030 | 0.040 | 0.040 | 0.008 | 0.040 | 0.040 | 0.030 |
| | Water-soluble polymer | PVP (polyvinylpyrrolidone) | 0.5 | 1.0 | 5.0 | 10.0 | 1.0 | 1.0 | 12.0 | 1.0 | 3.0 | 0.25 | - | 15.0 | - |
| | Water | Ion-exchanged water | 18.0 | 18.0 | 18.0 | 18.0 | 26.0 | 28.0 | 28.0 | 18.0 | 17.3 | 20.0 | 18.0 | 18.0 | 28.0 |
| | Moisturizing agent | Polyhydric alcohol | 58.0 | 57.5 | 53.5 | 48.5 | 51.7 | 45.6 | 34.7 | 57.5 | 44.0 | 55.8 | 58.5 | 43.5 | 45.7 |
| | Electrolyte salt | Sodium choride | 2.5 | 2.5 | 2.5 | 2.5 | 2.0 | 5.6 | 5.6 | 2.5 | 0.5 | 3.0 | 2.5 | 2.5 | 5.6 |
| | Amphiphilic polymer | | - | - | - | - | 0.5 | - | - | - | - | - | - | - | 1.0 |
| | 50% NaOH | | - | - | - | - | - | - | - | - | 8.0 | - | - | - | - |
| | Other additives | | 0.96 | 0.96 | 0.96 | 0.96 | 0.76 | 0.77 | 0.67 | 0.96 | 3.16 | 0.942 | 0.96 | 0.96 | 0.67 |
| Total | | | 100 | 100 | 100 | 100 | 100 | 100 | 100 | 100 | 100 | 100 | 100 | 100 | 100 |
| Thickness (mm) | | | 0.75 | 0.75 | 0.75 | 0.75 | 0.75 | 0.75 | 0.75 | 0.90 | 0.75 | 0.75 | 0.75 | 0.75 | 0.75 |

| | | Example | | | | | | | | Comparative Example | | | | |
|---|---|---|---|---|---|---|---|---|---|---|---|---|---|---|
| | | 1 | 2 | 3 | 4 | 5 | 6 | 7 | 8 | 1 | 2 | 3 | 4 | 5 |
| Evaluation | Conductivity (impedance $\Omega$) 1 kHz | 66 | 69 | 63 | 71 | 55 | 20 | 16 | 63 | 51 | 60 | 57 | - | 21 |
| | Conductivity (impedance $\Omega$)10Hz | 457 | 422 | 498 | 501 | 460 | 353 | 338 | 441 | 482 | 425 | 460 | - | 331 |
| | Mixture iquid solubility | Yes | Yes | Yes | Yes | Yes | Yes | Yes | Yes | Yes | Yes | Yes | No | Yes |
| | Adhesive force to Bakelite(N/20mm) | 4.5 | 4.8 | 4.7 | 4.8 | 3.9 | 3.0 | 3.1 | 5.8 | 4.8 | 3.6 | 4.4 | - | 3.0 |
| | Odor | No | No | No | No | No | No | No | No | Yes | No | No | - | No |
| | Gel tearing | Pass | Pass | Pass | Pass | Pass | Pass | Pass | Pass | Fail | Fail | Fail | - | Fail |
| | Tensile strength (N/20mm) | 0.7 | 0.9 | 1.0 | 1.1 | 0.8 | 0.7 | 1.2 | 0.9 | 0.4 | 0.2 | 0.4 | - | 0.2 |
| | Hardness(Pa) (storage modulus G) | 4129 | 4456 | 4876 | 5122 | 3500 | 3452 | 4259 | 4118 | 4028 | 2987 | 3874 | - | 3095 |
| | Adhesion in repeated use (I)Initial adhesion to skin (N/20mm) | 2.50 | 2.40 | 2.02 | 2.20 | 1.98 | 1.53 | 1.41 | 2.70 | 1.38 | 1.61 | 2.00 | - | 1.52 |
| | (II)Adhesion to skin after 20 application (N/20mm) | 2.31 | 2.25 | 1.81 | 1.88 | 1.77 | 1.41 | 1.17 | 2.38 | 1.28 | 1.22 | 1.38 | - | 1.01 |
| | (II)/(I) | A 0.94 | A 0.94 | A 0.90 | A 0.85 | A 0.89 | A 0.92 | A 0.83 | A 0.88 | A 0.93 | B 0.76 | C 0.69 | - | C 0.66 |

**Claims**

1.  A hydrogel comprising:

    a polymer matrix formed from a copolymer of an acrylic monomer and a crosslinkable monomer;
    a water-soluble polymer;
    water; and
    a polyhydric alcohol,
    the hydrogel having a tensile strength of 0.5 to 3.0 N/20 mm.

2.  The hydrogel according to claim 1, wherein the water-soluble polymer is polyvinylpyrrolidone.

3.  The hydrogel according to claim 1 or 2, wherein the acrylic monomer is an acrylamide-based monomer.

4.  The hydrogel according to any one of claims 1 to 3, wherein the content of a structural unit derived from the crosslinkable monomer is 0.01 mass% to 0.1 mass%.

5.  The hydrogel according to any one of claims 1 to 3, wherein the content of the water-soluble polymer is 0.1 mass% to 13 mass%.

6.  The hydrogel according to any one of claims 1 to 5, wherein an intermediate substrate is embedded along an in-plane direction.

7.  The hydrogel according to claim 6, wherein the intermediate substrate is a nonwoven fabric.

8.  The hydrogel according to any one of claims 1 to 7, which has a thickness of 0.2 mm to 2.0 mm.

9.  The hydrogel according to any one of claims 1 to 8, which has a storage modulus of 2000 Pa to 9000 Pa.

10. A medical electrode hydrogel to be placed between an electrode comprising a conductive material and a skin surface, the medical electrode hydrogel comprising the hydrogel according to any one of claims 1 to 9.

11. A method for producing the hydrogel according to any one of claims 1 to 9, comprising irradiating a polymerizable solution with ultraviolet light having a peak intensity of 70 to 150 mW/cm$^2$, the polymerizable solution comprising an acrylic monomer, a crosslinkable monomer, a water-soluble polymer, water, and a polyhydric alcohol and having a crosslinkable monomer content of 0.03 mass% to 0.05 mass%.

Fig. 1

(A)

(B)

## INTERNATIONAL SEARCH REPORT

| International application No. |
|---|
| PCT/JP2020/027635 |

**A.  CLASSIFICATION OF SUBJECT MATTER**
Int. Cl. A61Q19/00(2006.01)i, C08K5/053(2006.01)i, C08K7/02(2006.01)i, C08L33/26(2006.01)i, C08L39/06(2006.01)i, C08F2/44(2006.01)i, A61K9/70(2006.01)i, A61K8/04(2006.01)i, A61K8/34(2006.01)i, A61K8/81(2006.01)i
FI: C08L33/26, C08K5/053, C08L39/06, C08K7/02, C08F2/44 Z, A61K8/04, A61K8/81, A61K8/34, A61Q19/00, A61K9/70 405

According to International Patent Classification (IPC) or to both national classification and IPC

**B.  FIELDS SEARCHED**

Minimum documentation searched (classification system followed by classification symbols)
Int. Cl. A61Q19/00, C08K5/053, C08K7/02, C08L33/26, C08L39/06, C08F2/44, A61K9/70, A61K8/04, A61K8/34, A61K8/81

Documentation searched other than minimum documentation to the extent that such documents are included in the fields searched
Published examined utility model applications of Japan    1922-1996
Published unexamined utility model applications of Japan  1971-2020
Registered utility model specifications of Japan          1996-2020
Published registered utility model applications of Japan  1994-2020

Electronic data base consulted during the international search (name of data base and, where practicable, search terms used)

**C.  DOCUMENTS CONSIDERED TO BE RELEVANT**

| Category* | Citation of document, with indication, where appropriate, of the relevant passages | Relevant to claim No. |
|---|---|---|
| X<br>Y | JP 2003-96431 A (SEKISUI PLASTICS CO., LTD.) 03 April 2003, claims, paragraphs [0001], [0028]-[0044], example 3, claims, paragraphs [0001], [0028]-[0044], example 3 | 1-5, 8-11<br>6-7 |
| X<br>Y | JP 2012-153884 A (SEKISUI PLASTICS CO., LTD.) 16 August 2012, claims, paragraphs [0011], [0092]-[0112], example 1, claims, paragraphs [0011], [0092]-[0112], example 1 | 1-5, 8-11<br>6-7 |

☒  Further documents are listed in the continuation of Box C.        ☒  See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 09.09.2020 | 24.09.2020 |

| Name and mailing address of the ISA/<br>    Japan Patent Office<br>    3-4-3, Kasumigaseki, Chiyoda-ku,<br>    Tokyo 100-8915, Japan | Authorized officer<br><br>Telephone No. |
|---|---|

Form PCT/ISA/210 (second sheet) (January 2015)

**INTERNATIONAL SEARCH REPORT**

| International application No. |
| --- |
| PCT/JP2020/027635 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X<br>Y | JP 2012-144581 A (SEKISUI PLASTICS CO., LTD.) 02 August 2012, claims, paragraphs [0001], [0008], [0062]-[0080], example 4, claims, paragraphs [0001], [0008], [0062]-[0080], example 4 | 1-5, 8-11<br>6-7 |
| X<br>Y | JP 2012-62354 A (SEKISUI PLASTICS CO., LTD.) 29 March 2012, claims, paragraphs [0003], [0008], [0059]-[0078], example 1, claims, paragraphs [0003], [0008], [0059]-[0078], example 1 | 1-5, 8-11<br>6-7 |
| X<br>Y<br>A | WO 2018/062029 A1 (SEKISUI PLASTICS CO., LTD.) 05 April 2018, claims, paragraphs [0001], [0065]-[0094], example 6, claims, paragraphs [0001], [0065]-[0094], example 6, claims, paragraphs [0001], [0065]-[0094], example 6 | 1, 3-8, 10-11<br>6-7<br>2, 9 |

Form PCT/ISA/210 (continuation of second sheet) (January 2015)

**INTERNATIONAL SEARCH REPORT**
Information on patent family members

International application No.

PCT/JP2020/027635

| Patent Documents referred to in the Report | Publication Date | Patent Family | Publication Date |
|---|---|---|---|
| JP 2003-96431 A | 03.04.2003 | (Family: none) | |
| JP 2012-153884 A | 16.08.2012 | US 2013/0289157 A1 claims, paragraphs [0012], [0075]- [0088], example 1 EP 2799507 A1 CN 10402431 A KR 10-2013-0119961 A | |
| JP 2012-144581 A | 02.08.2012 | (Family: none) | |
| JP 2012-62354 A | 29.03.2012 | (Family: none) | |
| WO 2018/062029 A1 | 05.04.2018 | US 2019/0313931 A1 claims, paragraphs [0001], [0073]- [0102], example 6 EP 3520692 A1 KR 10-2019-0028802 A CN 109661198 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012107120 A
- JP 2003096431 A
- JP 2803886 B